# EUROPEAN PATENT SPECIFICATION

(11) **EP 1 397 348 B1**
(45) Date of publication and mention of the grant of the patent: **28.09.2005**
(21) Application number: 02778933.8
(22) Date of filing: 06.06.2002
(51) Int. Cl.: C07D 209/08, A61K 31/404, A61P 7/02

(54) **FACTOR XA INHIBITOR**
FAKTOR XA INHIBITOR
INHIBITEUR DU FACTEUR XA

(30) Priority: 12.06.2001 WO PCT/GB01/02553; 12.12.2001 US 339295 P
(43) Date of publication of application: 17.03.2004
(73) Proprietor: ELI LILLY AND COMPANY, Indianapolis, Indiana 46285 (US)
(72) Inventor: ENGEL, Gary, Lowell, Greenwood, IN 46143 (US); DISEROAD, Benjamin, Alan, Martinsville, IN 46151 (US)
(74) Representative: Hay, Martin Alexander
(86) International application number: PCT/US2002/016569
(87) International publication number: WO 2002/100847

(56) References cited:
- WO-A-00/76971
- WO-A-01/96323

## Description

This invention relates to a pharmaceutical compound that is a selective inhibitor of the serine protease, Factor Xa, to pharmaceutical compositions thereof and to its use in the treatment of the human or animal body.

The serine proteases are a group of proteolytic enzymes which have a common catalytic mechanism characterized by a particularly reactive Ser residue. Examples of serine proteases include trypsin, tryptase, chymotrypsin, elastase, thrombin, plasmin, kallikrein, Complement C1, acrosomal protease, lysosomal protease, cocoonase, α-lytic protease, protease A, protease B, serine carboxypeptidase II, subtilisin, urokinase, Factor VIIa, Factor IXa, and Factor Xa. The serine proteases have been investigated extensively over a period of several decades and the therapeutic value of inhibitors of serine proteases is well understood. Thus, for example, an inhibitor of Factor Xa has value as a therapeutic agent as an anticoagulant, e.g. in the treatment and prevention of thrombotic disorders. The use of a Factor Xa inhibitor as an anticoagulant is desirable in view of the selectivity of its effect. Many clinically approved anticoagulants have been associated with adverse events owing to the non-specific nature of their effects on the coagulation cascade.

It has been reported in WO99/11658 and WO99/11657 that certain benzamidine and aminoisoquinoline derivatives carrying a bulky lipophilic side chain are excellent inhibitors of serine proteases, in particular Factor Xa.

On 21 December, 2000, another series of serine protease inhibitors was disclosed in WO 00/76971. These compounds possess a variety of aromatic groups, such as indolyl, in place of the benzamidine and aminoisoquinoline groups found in the compounds of WO99/11658 and WO99/11657. One of the compounds specifically disclosed, in Example 318, was 1-(indole-6-carbonyl-D-phenylglycinyl)-4-(1-methylpiperidin-4-yl)piperazine.

1-(Indole-6-carbonyl-D-phenylglycinyl)-4-(1-methylpiperidin-4-yl)piperazine has been found to be a potent and selective inhibitor of Factor Xa, to have good oral exposure and to possess a particularly desirable pharmacological/ toxicological profile. The compound and its pharmaceutically acceptable salts are therefore potentially useful for the prophylaxis or treatment of thrombotic disorders such as amongst others venous thrombosis, pulmonary embolism, arterial thrombosis, myocardial ischaemia, myocardial infarction, and cerebral thrombosis. They also potentially have benefit in the treatment of acute vessel closure associated with thrombolytic therapy and restenosis, e.g. after transluminal coronary angioplasty or bypass grafting of the coronary or peripheral arteries and in the maintenance of vascular access patency in long term hemodialysis patients.

In order to be considered as a candidate for further development as a pharmaceutical, a compound must not only possess desirable biological properties, but also physical properties that adapt it for use in the manufacture of a pharmaceutical product. In particular, the compound should form a stable, preferably crystalline, solid that can readily be manufactured and formulated.

It has been found that the compound disclosed in Example 318 of WO 00/76971, 1-(indole-6-carbonyl-D-phenylglycinyl)-4-(1-methylpiperidin-4-yl)piperazine, can be obtained in crystalline form by crystallization from a chloroform/ethyl acetate solvent system. The crystalline form was found to be a chloroform solvate. Unfortunately, it proved difficult to remove the chloroform, which is undesirable in a pharmaceutical product, and in the presence of water, the crystalline material tended to convert into a gel.

The mono hydrochloride salt of 1-(indole-6-carbonyl-D-phenylglycinyl)-4-(1-methylpiperidin-4-yl)piperazine has also been prepared. The monohydrochloride salt was initially obtained as an amorphous solid. This solid was cycled through a vapor pressure isotherm determination, in which the solid initially deliquesced, then dehydrated. The dehydrated material was found to be crystalline. Unfortunately the crystalline material, like the amorphous material, was found to be hygroscopic.

Surprisingly, a stable, crystalline form of a pharmaceutically acceptable salt of 1-(indole-6-carbonyl-D-phenylglycinyl)-4-(1-methylpiperidin-4-yl)piperazine has now been found. It was found by dissolving 1-(indole-6-carbonyl-D-phenylglycinyl)-4-(1-methylpiperidin-4-yl)piperazine in 95% ethanol at about 50 °C, adding two equivalents of fumaric acid and allowing the resultant solution to cool.

Thus viewed from one aspect the invention provides 1-(indole-6-carbonyl-D-phenylglycinyl)-4-(1-methylpiperidin-4-yl)piperazine difumarate.

An alternative name by which the compound may be known is 1H-indole-6-carboxamide, N-[(1R)-2-[4-(1-methyl-4-piperidinyl)-1-piperazinyl]-2-oxo-1-phenylethyl]-, (2E)-butenedioate (1:2) (salt).

It will be appreciated that the compound may exist in racemic (D/L) or chiral form, and that the preferred D-isomer may be administered in a racemic mixture with the L-isomer, or alone. The D-conformation refers to the conformation of D-phenylglycine, from which the compound may be prepared.

According to another aspect, the present invention provides 1-(indole-6-carbonyl-D-phenylglycinyl)-4-(1-methylpiperidin-4-yl)piperazine difumarate in crystalline form.

It has been found that the difumarate salt can be obtained in at least two different crystalline forms, depending upon the solvent system used to crystallize it.

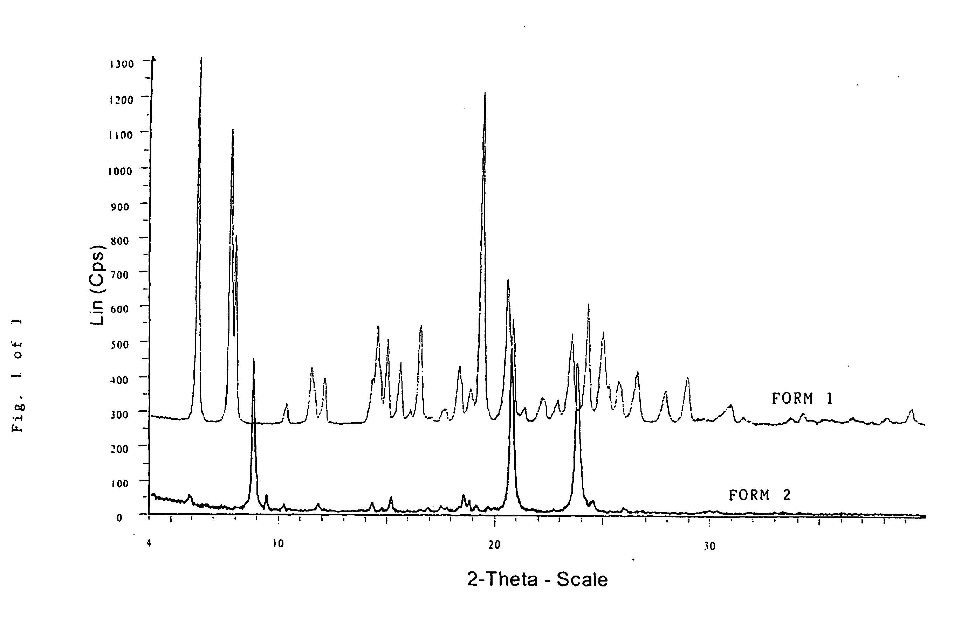

The first crystalline form of the difumarate salt has been prepared by dissolving the free base in methanol or 95% ethanol, warming the solution to about 50°C, adding two equivalents of fumaric acid in methanol or 95% ethanol, then allowing the resultant mixture to cool. A salt, identified to be the difumarate, was found to crystallize out as thin needles. Analysis by differential scanning calorimetry (DSC) revealed a sharp melting point at about 213°C. The crystalline material was subjected to X-ray powder diffraction analysis. The resultant X-ray powder diffraction pattern was found to contain sharp, intense peaks at 2θ = 6.280, 7.830 and 19.513. This crystalline form of the difumarate salt is hereinafter referred to as Form 1. A more detailed analysis of the peaks is provided in Table 1 below. The X-ray powder diffraction pattern is shown in Figure 1.

It has been found that Form 1 can readily be filtered off, and is morphologically stable, even under conditions of high relative humidity (above 70%). The results of the stability studies are tabulated in Table 2 below.

**Table 1**

| | | Intensity |
|---|---|---|
| Angle | d value | % |
| 2-Theta ° | Angstrom | % |
| 6.28 | 14.06278 | 100 |
| 7.83 | 11.2814 | 80.9 |
| 8.047 | 10.97827 | 51.1 |
| 10.354 | 8.53661 | 5.4 |
| 11.577 | 7.63712 | 15.5 |
| 11.707 | 7.55314 | 10.1 |
| 12.146 | 7.28071 | 12.8 |
| 14.381 | 6.15405 | 12.1 |
| 14.599 | 6.06232 | 25.8 |
| 15.087 | 5.86757 | 22.3 |
| 15.647 | 5.65872 | 16.1 |
| 16.135 | 5.48865 | 3.2 |
| 16.61 | 5.33267 | 26.2 |
| 17.075 | 5.18873 | 1 |
| 17.705 | 5.00543 | 3.1 |
| 18.395 | 4.81901 | 15 |
| 18.928 | 4.68464 | 8.5 |
| 19.513 | 4.54547 | 88.1 |
| 20.672 | 4.29321 | 39 |
| 20.9 | 4.2469 | 28.2 |
| 21.354 | 4.15765 | 3.4 |
| 22.238 | 3.99418 | 5.9 |
| 22.899 | 3.88038 | 4.3 |
| 23.571 | 3.77122 | 23 |
| 24.328 | 3.65558 | 31.2 |
| 25.014 | 3.55687 | 22.9 |
| 25.311 | 3.51589 | 9.8 |
| 25.76 | 3.4556 | 10.5 |
| 26.602 | 3.34803 | 14.1 |
| 27.915 | 3.19351 | 8.3 |
| 28.939 | 3.08279 | 12.2 |
| 30.94 | 2.88781 | 4.6 |
| 31.571 | 2.83149 | 1.8 |
| 33.738 | 2.65444 | 1.9 |
| 34.29 | 2.61297 | 2.8 |
| 35.275 | 2.54221 | 1 |
| 36.577 | 2.45468 | 1.4 |
| 38.214 | 2.35319 | 1.8 |
| 39.326 | 2.28917 | 3.9 |

**Table 2**

| **Stability study on Form 1** | | |
|---|---|---|
| **Condition** | **7 Days** | **28 Days** |
| 40 °C | 100.5 | 99.8 |
| 50 °C | 99.3 | 99.4 |
| 70 °C | 99.0 | 100.2 |
| 40 °C/75% RH | 99.6 | 99.5 |
| Light | 99.7 | 98.9 |
| Notes: % expressed with reference to material stored at -70 °C. % RH means percentage relative humidity. | | |

The second crystalline form of the difumarate salt was prepared by crystallization from 50% aqueous ethanol. The crystalline material was subjected to X-ray powder diffraction analysis. The resultant X-ray powder diffraction pattern was found to contain sharp, intense peaks at 2θ = 8.833, 20.810 and 23.824. This crystalline form of the difumarate salt is hereinafter referred to as Form 2. A more detailed analysis of the peaks is provided in Table 3 below. The X-ray powder diffraction pattern is shown in Figure 1.

It has been found that Form 2 is morphologically stable, even under conditions of high relative humidity (above 70%). The results of the stability studies are tabulated in Table 4 below.

After Form 2 had been prepared by crystallization from from 50% aqueous ethanol, it was found that this form could also be prepared by suspending Form 1 in water, and by crystallization from water. Stability in the presence of water is desirable in a product intended to be formulated in a pharmaceutical composition using a process that brings it into contact with water, such as a wet granulation process used in the preparation of a tablet.

**Table 3**

| Angle d 2-Theta | value ° Angstrom | Intensity % |
|---|---|---|
| 5.843 | 15.11276 | 5.6 |
| 8.833 | 10.0024 | 92.5 |
| 9.436 | 9.36541 | 9.5 |
| 10.226 | 8.64333 | 4.4 |
| 11.807 | 7.4888 | 4.9 |
| 14.312 | 6.18352 | 5.5 |
| 14.745 | 6.00264 | 2.5 |
| 15.207 | 5.82159 | 9.5 |
| 16.94 | 5.22959 | 2.5 |
| 17.54 | 5.05196 | 3.4 |
| 17.815 | 4.97472 | 2.6 |
| 18.575 | 10.2 | 10.2 |
| 18.825 | 4.71005 | 6.7 |
| 19.144 | 4.63231 | 3.7 |
| 19.708 | 4.50099 | 2.7 |
| 20.81 | 4.26508 | 100 |
| 23.824 | 3.73184 | 90.9 |
| 24.507 | 3.62937 | 7.3 |
| 25.986 | 3.42603 | 3.3 |
| 30.314 | 2.94599 | 1.9 |
| 31.832 | 2.80888 | 1.4 |
| 33.025 | 2.71013 | 1.6 |
| 33.366 | 2.68322 | 1.5 |
| 36.106 | 2.48562 | 1.3 |

**Table 4**

| **Stability study on Form 2** | | |
|---|---|---|
| **Condition** | **7 Days** | **28 Days** |
| 70 °C | 99.1 | 99.0 |
| 40 °C/75% RH | 99.3 | 99.6 |
| Notes: % expressed with reference to material stored at -70 °C. % RH means percentage relative humidity. | | |

X-ray powder diffraction patterns were obtained on a Siemens D5000 X-ray diffractomenter equipped with a CuK source (λ = 1.54056) operating at a tube load of 50 KV and 40 mA. The divergence slit size was 1 mm, the receiving slit 1 mm, and the detector slit 0.1 mm. Data were collected by a Kevex solid-state (SiLi) detector. Each sample was scanned between 4 and 35 degrees (2-theta) with a step size of 0.02 degrees and a maximum scan rate of 3 sec/step.

It will be appreciated by those skilled in the art of X-ray powder diffraction analysis that the exact values measured for 2θ (or the corresponding d-spacings) may vary depending upon the particular sample analysed and the apparatus and particular analysis procedure used.

1-(Indole-6-carbonyl-D-phenylglycinyl)-4-(1-methylpiperidin-4-yl)piperazine may be prepared by the method described in WO 00/76971 or as described in the following examples.

It will be understood that the difumarate salt according to the invention may be isolated in the form of solvates (which may or may not be physiologically tolerable), and that all such solvates are therefore included within the scope of the present invention. It will be appreciated that a solvate that is not physiologically tolerable may nevertheless be useful in the manufacture of a pharmaceutical product, for example in a purification step.

The difumarate salt of the invention may be administered by any convenient route, e.g. into the gastrointestinal tract (e.g. rectally or orally), the nose, lungs, musculature or vasculature or transdermally. The difumarate salt may be administered in any convenient administrative form, e.g. tablets, powders, capsules, solutions, dispersions, suspensions, syrups, sprays, suppositories, gels, emulsions, patches etc. Such compositions may contain components conventional in pharmaceutical preparations, e.g. diluents, carriers, pH modifiers, sweeteners, bulking agents, and further active agents. Preferably, for injection or infusion, the compositions will be sterile and in a suitable solution or suspension form. Such compositions form a further aspect of the invention.

Viewed from this aspect the invention provides a pharmaceutical composition comprising the difumarate salt according to the invention together with at least one pharmaceutically acceptable carrier or excipient. The pharmaceutical composition may also optionally comprise at least one further antithrombotic and/or thrombolytic agent. The compound may, with benefit, form part of a combination therapy with an anticoagulant with a different mode of action or with a thrombolytic agent.

Viewed from a further aspect the invention provides the use of the difumarate salt according to the invention for the manufacture of a medicament for use in a method of treatment of the human or non-human animal body (e.g. a mammalian, avian or reptilian body) to combat (i.e. treat or prevent) a condition responsive to said inhibitor.

The dosage of the compound of the invention will depend upon the nature and severity of the condition being treated, the administration route and the size and species of the patient. However in general, quantities of from 0.01 to 100 µmol/kg bodyweight will be administered.

The invention will now be described further with reference to the following non-limiting Examples.

### Experimental

Abbreviations used follow IUPAC-IUB nomenclature. The following abbreviations are used throughout: Boc (tertiary-butyloxycarbonyl), MeOH (methanol), API-MS (ion spray mass spectrum), THF (tetrahydrofuran), DSC (differential scanning calorimetry) and TGA (thermal gravimetric analysis).

All solution concentrations are expressed as %Vol./%Vol. unless otherwise stated. Reagents were obtained from a variety of commercial sources.

IR means an infrared spectrum was obtained. ¹NMR, NMR, 1H-NMR, or 1H NMR means a proton magnetic resonance spectrum was obtained.
API-MS (atmospheric pressure chemical ionization mass spectra) were obtained on a PESciex API 150EX with a heated nebulizer and nitrogen as the reagent gas in positive ion mode.

### Example 1

### Preparation of Form 1 of 1-(Indole-6-carbonyl-D-phenylglycinyl)-4-(1-methylpiperidin-4-yl)piperazine Difumarate.

The difumarate salt is conveniently prepared by dissolving the free base in methanol or 95% ethanol and warming to about 50 °C (for example at a concentration of 460 mg in 15 mL). Two molar equivalents of fumaric acid (for example 232.2 mg) are then added (for example, as a 0.25 M solution in methanol or as a suspension in 3 mL 95% ethanol). Following cooling and crystallization, and isolation and drying, the product is obtained as thin crystalline needles, with a sharp melting point at about 213 °C by DSC.

### Example 2

### Preparation of Form 2 of 1-(Indole-6-carbonyl-D-phenylglycinyl)-4-(1-methylpiperidin-4-yl)piperazine Difumarate.

The difumarate salt (15mg) is dissolved in 50% aqueous ethanol (EtOH:H₂O/50:50) using sonication to aid dissolution. The solvent is then allowed to evaporate at ambient temperature overnight.

### Example 2a

### Preparation of Form 2 by Suspension of Form 1 in Water.

1.5 g of Form 1 of 1-(indole-6-carbonyl-D-phenylglycinyl)-4-(1-methylpiperidin-4-yl)piperazine difumarate was suspended in 10 ml of distilled water and stirred for 24 hours. The suspension thickened after 5-6 hours, but stirring was continued for the remaining time. The suspension was centrifuged to isolate the solid. The solid was washed with cyclohexane and dried at ambient temperature under vacuum. Analysis of the product by X-ray powder diffraction showed that the solid was no longer Form 1 and had converted into Form 2.

### Example 2b

### Preparation of Form 2 by Crystallization from Water.

2.5 g of 1-(indole-6-carbonyl-D-phenylglycinyl)-4-(1-methylpiperidin-4-yl)piperazine difumarate was suspended in 25 ml of water. The suspension was then heated to about 60-70 °C and stirred until a clear solution was obtained. The clear solution was then allowed to cool to ambient temperature overnight, with stirring. The next day, crystals were collected by filtration and dried under vacuum at 40 °C.

### Preparation of Intermediates

1-(Indole-6-carbonyl-D-phenylglycinyl)-4-(1-methylpiperidin-4-yl)piperazine may be prepared by the method of Example 318 of WO 00/76971. Alternatively it may be prepared from Boc-D-Phg-OH and 1-(1-methylpiperidin-4-yl)piperazine as described hereinbelow.

### Intermediate 1

### 1-Boc-D-Phenylglycinyl-4-(1-methylpiperidin-4-yl)piperazine.

Boc-D-Phg-OH (40.0 g, 159.2 mmol) and 1-(1-methyl-piperidin-4-yl)piperazine (32.1 g, 175.1 mmol) were slurried in anhydrous dichloromethane (1.5 L) under N₂. The mixture was then cooled to -15 °C in an ice/MeOH bath. Triethylamine (26.6 mL, 191.0 mmol) was added slowly, maintaining the temperature at -15 °C, followed by slow addition of diethyl cyanophosphonate (29.0 mL, 191.0 mmol), again maintaining temp at -15 °C. The reaction mixture was allowed to warm to room temperature overnight. The reaction was then quenched with the addition of satd NaHCO₃ (500 mL), and the layers were separated. The aqueous layer was then extracted with dichloromethane (3 x 1 L). The organic layers were combined, dried over Na₂SO₄, filtered and concentrated in vacuo to give a crude oil. Purification using (Biotage) Flash Chromatography with 7.5% (2 M NH₃ in MeOH) in THF gave 53.6 g (81%) of the title compound.
¹H NMR (DMSO-_{d6}) δ 7.33 (m, 5 H), 7.12 (d, J = 8.1 Hz, 1 H), 5.53 (d, J = 8.1 Hz, 1 H), 3.31 (m, 5 H), 2.72 (d, J = 11.3 Hz, 2 H), 2.3 (m, 3 H), 2.09 (s, 3 H), 2.03 - 1.86 (m, 2 H), 1.76 (dt, J = 9.7, 1.8 Hz, 2 H), 1.56 (m, 2 H), 1.36 (s, 9 H).
IS-MS, m/e 416.27 (M+1).
Chiral HPLC indicated no racemization had occurred.

### Intermediate 2

### 1-D-Phenylglycinyl-4-(1-methylpiperidin-4-yl)piperazine Trihydrochloride.

1-Boc-D-phenylglycinyl-4-(1-methylpiperidin-4-yl)-piperazine (49.6 g, 119.1 mmol) was dissolved in anhydrous MeOH (1 L) and HCl (gas) was bubbled through the solution for 2 h 15 min, noting the formation of a white precipitate. The solvents were removed in vacuo to give 48.3 g (95%) of the title compound as an off-white foam.
¹H NMR (DMSO-d₆) δ 12.08 (bs, 1 H), 11.03 (bs, 1 H), 8.92 (bs, 2 H), 8.79 (bs, 1 H), 7.54 (m, 2 H), 7.47 (m, 3 H), 5.66 (s, 1 H), 4.49 (m, 1 H), 4.26 (bd, 1 H), 3.91 (bs, 2 H), 3.5 - 2.8 (m, 9 H), 2.69 (s, 3 H), 2.4 - 1.8 (m, 4 H). IS-MS, m/e 316.24 (M+1).

### Intermediate 3

### 1-(Indole-6-carbonyl-D-phenylglycinyl)-4-(1-methylpiperidin-4-yl)piperazine.

Indole-6-carboxylic acid (16.0 g, 99.3 mmol) and 1-D-phenylglycinyl-4-(1-methylpiperidin-4-yl)piperazine trihydrochloride (42.3 g, 99.3 mmol) were slurried in anhydrous dichloromethane (1 L) under N₂. The mixture was then cooled to -15 °C in an ice/MeOH bath. Triethylamine (58.1 mL, 416.9 mmol) was added slowly, maintaining the temperature at -15 °C, followed by slow addition of diethyl cyanophosphonate (18.1 mL, 119.1 mmol), maintaining the temperature at -15 °C. The reaction mixture was allowed to warm to room temperature overnight. The reaction was then quenched with the addition of satd NaHCO₃ (500 mL), and the layers were separated. The aqueous layer was then extracted with dichloromethane (3 x 500 mL). The organic layers were combined, dried over Na₂SO₄, filtered and concentrated to give a crude oil. Purification was performed using (Biotage) Flash Chromatography, eluting with 8.3% (2 M NH₃ in MeOH) in CHCl₃. The product containing fractions were combined and concentrated in vacuo to give 45.1 g (99%) of the title compound.
¹H NMR (DMSO-d₆) δ 11.35 (s, 1 H), 8.65 (d, J = 7.7 Hz, 1 H), 7.98 (s, 1 H), 7.60 - 7.45 (m, 5 H), 7.40 - 7.25 (m, 3 H), 6.48 (t, J = 2.0 Hz, 1 H), 6.09 (d, J = 7.7 Hz, 1 H), 3.5 (m, 3 H), 2.72 (d, J = 11.3 Hz, 2 H), 2.40 (m, 2 H), 2.09 (s, 3 H), 2.05 (m, 2 H), 1.77 (dt, J = 1.1, 10.2 Hz, 2 H), 1.59 (d, J = 11.3 Hz, 2 H), 1.31 (m, 2 H).
¹³C NMR (DMSO-d₆) δ 168.0, 166.4, 138.0, 135.1, 129.9, 128.4, 128.2, 128.0, 127.6, 126.6, 119.4, 118.1, 111.5, 101.2, 79.1, 60.6, 54.7, 53.7, 48.5, 48.3, 45.8, 45.4, 42.2, 27.7, 27.6.
IS-MS, m/e 459.26 (M+1).
[α]_{D}²⁰ = -73.08 (c=0.02, MeOH).

A portion of the free base was isolated from a chloroform - ethyl acetate solvent system as crystalline material which was birefringent by microscopy. From DSC and TGA, the material was found to be a solvate containing 0.5 mol chloroform per mol of free base. The chloroform solvate was found to have a broad endotherm about 148-158 °C, followed by a sharper endotherm (peak at 194.4 °C) as the melting point of the desolvated free base.

### Comparative Example 1

### 1-(Indole-6-carbonyl-D-phenylglycinyl)-4-(1-methylpiperidin-4-yl)piperazine Hydrochloride.

To a solution of 1-(indole-6-carbonyl-D-phenylglycinyl)-4-(1-methylpiperidin-4-yl)piperazine (14.5 g, 31.6 mmol) in anhydrous dichloromethane (300 mL) and anhydrous MeOH (150 mL) at 0 °C was added HCl in Et₂O (32.2 mL, 32.2 mmol). After approximately 5 min, the solvents were removed in vacuo to give 15.1 g (96%) of the title compound.
¹H NMR (DMSO-d₆) δ 11.40 (s, 1 H), 10.3 (bs, 1 H), 8.68 (m, 1 H), 7.99 (s, 1 H), 7.6 - 7.4 (m, 5 H), 7.4 - 7.3 (m, 3 H), 6.48 (s, 1 H), 6.11 (d, J = 7.3 Hz, 1 H), 4.08 (bs, 1 H), 3.6 - 1.5 (bm, 15 H), 2.66 (s, 3 H).
IS-MS, m/e 459.26 (M+1).
[α]_{D}²⁰ = -83.67 (c=0.01, MeOH).

| Analysis for C₂₇H₃₃N₅O₂·1.1 HCl·1.7 H₂O: | | | | |
|---|---|---|---|---|
| Calcd | C, 61.03; | H, 7.30; | N, 13.18; | Cl, 7.34; |
| Found | C, 60.95; | H, 6.91; | N, 13.03; | Cl, 7.00. |

The product prepared by the above method was found to be the mono-hydrochloride salt and to be amorphous. Analysis by microscopy showed glassy non-birefringent particles; and analysis by DSC failed to reveal a melting point, in agreement with amorphous material. Using a microbalance flow system, the original material was cycled through a vapor pressure isotherm determination, where the material deliquesced, then allowed to deydrate. Upon dehydration, there were formed crystals which were birefringent by microscopy; and a melting point of about 174 °C was demonstrated for the newly crystallized, hygroscopic material.

## Claims

1. 1-(Indole-6-carbonyl-D-phenylglycinyl)-4-(1-methylpiperidin-4-yl)piperazine Difumarate.

2. 1-(Indole-6-carbonyl-D-phenylglycinyl)-4-(1-methylpiperidin-4-yl)piperazine Difumarate in crystalline form.

3. 1-(Indole-6-carbonyl-D-phenylglycinyl)-4-(1-methylpiperidin-4-yl)piperazine Difumarate in crystalline form having an X-ray powder diffraction pattern with sharp, intense peaks at 2θ = 6.280, 7.830 and 19.513.

4. 1-(Indole-6-carbonyl-D-phenylglycinyl)-4-(1-methylpiperidin-4-yl)piperazine Difumarate in crystalline form having an X-ray powder diffraction pattern with sharp, intense peaks at 2θ = 8.833, 20.810 and 23.824.

5. A pharmaceutical composition, which comprises the difumarate salt as claimed in any one of Claims 1 to 4 together with at least one pharmaceutically acceptable carrier or excipient.

6. The difumarate salt as claimed in any one of Claims 1 to 4, for use in therapy.

7. Use of the difumarate salt as claimed in any one of Claims 1 to 4 for the manufacture of a medicament for the treatment of a thrombotic disorder.

8. A pharmaceutical composition comprising the difumarate salt as claimed in any one of Claims 1 to 4 for use to combat a thrombotic disorder.

## Patentansprüche

1. 1-(Indol-6-carbonyl-D-phenylglycinyl)-4-(1-methylpiperidin-4-yl)piperazindifumarat.

2. 1-(Indol-6-carbonyl-D-phenylglycinyl)-4-(1-methylpiperidin-4-yl)piperazindifumarat in kristalliner Form.

3. 1-(Indol-6-carbonyl-D-phenylglycinyl)-4-(1-methylpiperidin-4-yl)piperazindifumarat in kristalliner Form mit einem Röntgenpulverbeugungsbild mit scharfen, intensiven Peaks bei 2θ = 6,280; 7,830 und 19,513.

4. 1-(Indol-6-carbonyl-D-phenylglycinyl)-4-(1-methylpiperidin-4-yl)piperazindifumarat in kristalliner Form mit einem Röntgenpulverbeugungsbild mit scharfen, intensiven Peaks bei 2θ = 8,833; 20,810 und 23,824.

5. Pharmazeutische Zusammensetzung, die das Difumaratsalz, wie es in einem der Ansprüche 1 bis 4 beansprucht ist, zusammen mit mindestens einem pharmazeutisch annehmbaren Träger oder Hilfsstoff umfasst.

6. Das Difumaratsalz, wie in einem der Ansprüche 1 bis 4 beansprucht, zur Verwendung bei einer Therapie.

7. Verwendung von dem Difumaratsalz, wie in einem der Ansprüche 1 bis 4 beansprucht, zur Herstellung eines Arzneimittels für die Behandlung einer thrombotischen Störung.

8. Pharmazeutische Zusammensetzung, umfassend das Difumaratsalz, wie in einem der Ansprüche 1 bis 4 beansprucht, zur Verwendung, um eine thrombotische Störung zu bekämpfen.

## Revendications

1. Difumarate de 1-(indole-6-carbonyl-D-phénylglycinyl)-4-(1-méthylpipéridin-4-yl)pipérazine.

2. Difumarate de 1-(indole-6-carbonyl-D-phénylglycinyl)-4-(1-méthylpipéridin-4-yl)pipérazine sous forme cristalline.

3. Difumarate de 1-(indote-6-carbonyl-D-phénylglycinyl)-4-(1-méthylpipéridin-4-yl)pipérazine sous forme cristalline ayant un diagramme de diffraction aux rayons X des poudres avec des pics aigus, intenses à 2θ = 6,280, 7,830 et 19,513.

4. Difumarate de 1-(indole-6-carbonyl-D-phénylglycinyl)-4-(1-méthylpipéridin-4-yl)pipérazine sous forme cristalline ayant un diagramme de diffraction aux rayons X des poudres avec des pics aigus, intenses à 2θ = 8,833, 20,810 et 23,824.

5. Composition pharmaceutique, qui comprend le sel difumarate comme revendiqué dans l'une quelconque des revendications 1 à 4 ensemble avec au moins un véhicule ou un excipient pharmaceutiquement acceptables.

6. Sel difumarate comme revendiqué dans l'une quelconque des revendications 1 à 4, pour une utilisation dans un traitement thérapeutique.

7. Utilisation du sel difumarate comme revendiqué dans l'une quelconque des revendications 1 à 4 pour la fabrication d'un médicament pour le traitement d'un trouble thrombotique.

8. Composition pharmaceutique comprenant le sel difumarate comme revendiqué dans l'une quelconque des revendications 1 à 4 pour une utilisation pour combattre un trouble thrombotique.
